(19) Europäisches Patentamt
European Patent Office
Office européen des brevets

(11) EP 4 778 531 A1

(12) EUROPEAN PATENT APPLICATION
published in accordance with Art. 153(4) EPC

(43) Date of publication:
**22.07.2026 Bulletin 2026/30**

(21) Application number: **24864790.1**

(22) Date of filing: **14.09.2024**

(51) International Patent Classification (IPC):
***A61K 31/724*** *(2006.01)* ***A61K 9/19*** *(2006.01)*
***A61K 9/08*** *(2006.01)* ***A61P 21/00*** *(2006.01)*
***A61K 31/27*** *(2006.01)*

(52) Cooperative Patent Classification (CPC):
**A61K 9/08; A61K 9/19; A61K 31/27; A61K 31/715; A61K 31/724; A61K 45/06; A61P 21/00; A61P 21/02**

(86) International application number:
**PCT/CN2024/119212**

(87) International publication number:
**WO 2025/056075 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.09.2023 CN 202311194721**

(71) Applicant: **Hangzhou Adamerck Pharmlabs Inc.**
**Hangzhou, Zhejiang 311112 (CN)**

(72) Inventor: **QI, Youmao**
**Hangzhou, Zhejiang 311112 (CN)**

(74) Representative: **Mewburn Ellis LLP**
**Aurora Building**
**Counterslip**
**Bristol BS1 6BX (GB)**

# (54) MUSCLE RELAXANT ANTAGONIST FOR REVERSAL FOR BENZYLISOQUINOLINE MUSCLE RELAXANT

(57) The present invention provides a muscle relaxant antagonist for reversal for a benzylisoquinoline muscle relaxant. Specifically, the present invention provides a preparation method for a safer and more effective muscle relaxant antagonist pharmaceutical preparation for reversing a benzylisoquinoline muscle relaxant, and a use. More specifically, the present invention provides adamgammadex sodium serving as an alternative to neostigmine to reverse muscle relaxation by a benzylisoquinoline muscle relaxant cisatracurium besylate. During reversal of residual neuromuscular blockade after muscle relaxation induced by the muscle relaxant cisatracurium besylate, adamgammadex sodium can serve as an alternative to neostigmine, demonstrating significant superiority over neostigmine in terms of heart rate maintenance while maintaining comparable antagonist efficacy. The present invention also provides adamgammadex sodium and a compound pharmaceutical preparation thereof. Adamgammadex sodium is prepared into a freeze-dried powder injection or liquid injection and is applicable to the preparation of a muscle relaxant antagonist.

EP 4 778 531 A1

## Description

### TECHNICAL FIELD

The present invention relates to the field of medicine, and in particular, to a method for preparing a safer and more effective muscle relaxant antagonist pharmaceutical preparation for reversing benzylisoquinoline muscle relaxants (e.g., cisatracurium besylate) and use thereof.

### BACKGROUND

With the continuous development and advancement of surgery and minimally invasive medicine, and the increasing demand of patients for surgical comfort and safety, the proportion of general anesthesia in clinical surgical anesthesia is growing, reaching over 90% in some hospitals.

Muscle relaxants are an important component of general anesthetics. They provide surgeons with a clearer surgical field, ensure that patient does not move during surgery, and reduce the incidence of intraoperative complications. Cisatracurium is a relatively new intermediate-acting non-depolarizing muscle relaxant and one of the isomers of atracurium. It is primarily degraded by Hofmann elimination at physiological pH and body temperature, independent of hepatic or renal metabolism. Clinically, it is characterized by no accumulation, rapid recovery, and potent effect. Its particularly low histamine release and minimal impact on cardiovascular function have led to its widespread clinical use. Currently, the most commonly used clinical muscle relaxants are intermediate-acting non-depolarizing agents such as cisatracurium, rocuronium, and vecuronium, which together account for over 98% of the muscle relaxant market. Among these, cisatracurium holds the largest share and can be regarded as the leader in the muscle relaxant market.

Incomplete metabolism of muscle relaxants often leads to respiratory insufficiency in patients after extubation. Furthermore, residual neuromuscular blockade can impede the recovery of laryngeal muscle function, increasing the incidence of postoperative aspiration, dysphagia, and upper airway obstruction in patients. If the patient's respiration is suppressed, it can lead to hypoxemia. This residual muscle relaxation effect can increase the postoperative complications, mortality rate of patients, and prolong hospital treatment time for them. It has been reported in the literature that even in the United States and Canada, where neuromuscular monitoring is relatively commonly used nowadays, the incidence of residual muscle relaxation upon extubation after surgery reaches 64.7% and 63.5%, respectively. Additionally, it has been reported in the literature that when the non-depolarizing muscle relaxant such as cisatracurium is used according to the conventional method during general anesthesia, it was found that 12% of the patients still had the risk of residual muscle relaxation (TOFr < 0.9; TOFr: the ratio of the 4th muscle twitch to the 1st muscle twitch T4/T1) when the endotracheal tube was removed after the operation.

Currently, the commonly used muscle relaxant antagonists in clinical practice are anticholinesterase drugs, which function by inhibiting acetylcholinesterase, thereby reducing the breakdown of acetylcholine (ACh). The most commonly used anticholinesterase drug in clinical practice is neostigmine. While antagonizing the muscle relaxant effect, it also produces significant atropine-like effects, particularly on the heart rate, potentially inducing bradycardia, which in severe cases can lead to cardiac arrest. This leads to the need for many patients to avoid or use this drug with caution. Atropine is an anticholinergic drug that can increase heart rate and counteract the bradycardia induced by neostigmine. Clinically, these two drugs are often used in combination to counteract the residual muscle relaxation effect, and they are usually used together. However, since atropine takes effect quickly while neostigmine takes effect relatively slowly, there will be a clear process of increased heart rate at the beginning. This hemodynamic change can cause perioperative myocardial ischemia, and for some patients with more severe heart conditions, it can cause more serious effects or consequences. Some researchers suggest administering atropine 2-4 minutes after neostigmine, however, the anesthesia recovery room is very busy, making it difficult to ensure that the medication is administered at the prescribed time every time. If atropine is injected too late, it may pose a risk of excessive bradycardia. Furthermore, atropine can easily cross the blood-brain barrier and is a risk factor for postoperative cognitive dysfunction. It may even lead to delirium, and for elderly patients, the risk is extremely high.

Adamgammadex sodium is a safer alternative to neostigmine in the present invention as a muscle relaxant antagonist, used for reversing the muscle relaxant cisatracurium besilate. Adamgammadex sodium is a small-molecule drug developed by our company and is classified as a Class 1 Chemical Drug in China. Currently, the drug's highest developmental stage is Phase III clinical trials for the reversal of neuromuscular blockade.

Sugammadex sodium (trade name: Bridion), is the only currently registered product of its kind as adamgammadex sodium described in this invention. As a novel muscle relaxant antagonist, it is a specific antagonist for rocuronium (a steroidal muscle relaxant). Sugammadex sodium does not bind to succinylcholine or benzylisoquinoline-type neuromuscular blocking agents (such as mivacurium, atracurium, and cisatracurium. Instead, following administration of sugammadex sodium, the neuromuscular blockade induced by cisatracurium took effect more quickly and the degree of blockade was deeper. The earliest research reports on sugammadex sodium and atracurium was published in 2006 by H.

D. de Boer et al. in the British Journal of Anaesthesia. They found that sugammadex sodium was ineffective in reversing the neuromuscular blockade induced by atracurium, although it had no effect on blood pressure or heart rate. The 2023 American Society of Anesthesiologists Practice Guidelines for Monitoring and Antagonism of Neuromuscular Blockade also state that sugammadex sodium is ineffective for antagonizing benzylisoquinoline-type neuromuscular blocking drugs. Additionally, sugammadex sodium has certain safety concerns. It was rejected by the US FDA three times due to anaphylactic reactions, QT prolongation in the heart, and bleeding risk. It took 7 years before it was finally approved for market release. In recent years, there have been an increasing number of potential adverse reactions associated with succinylcholine sodium, including common and serious adverse reactions such as residual muscle relaxation after surgery, allergic reactions and hypersensitivity reactions, coagulation dysfunction, cardiovascular system side effects, bronchospasm and laryngeal spasm, as well as other adverse reactions such as postoperative nausea, prolonged renal excretion time, neuronal apoptosis and abnormal taste sensation with metallic taste. Therefore, cholinesterase inhibitors, and cholinesterase inhibitors combined with anticholinergic drugs, have not yet met clinical needs. There is an urgent need to provide patients with muscle relaxant antagonistic treatment drugs that are both effective in antagonizing muscle relaxation and safe.

## SUMMARY OF THE INVENTION

The purpose of the present invention is to provide a use of adamgammadex sodium in the preparation of a pharmaceutical composition for reversing neuromuscular blockade induced by benzylisoquinoline muscle relaxants (e.g., cisatracurium besylate).

In the first aspect of the present invention, provided is a use of adamgammadex sodium for the preparation of a pharmaceutical composition for reversing muscle relaxation induced by benzylisoquinoline muscle relaxants (e.g., cisatracurium besylate).

The use according to the first aspect of the present invention, wherein, the pharmaceutical composition is used to reverse residual neuromuscular blockade after muscle relaxation induced by cisatracurium.

The use according to the first aspect of the present invention, wherein, the pharmaceutical composition is also used to alleviate the adverse reactions during the reversal of muscle relaxation induced by cisatracurium. The adverse reactions are selected from the group consisting of: decreased heart rate, decreased blood pressure, and gastrointestinal discomfort.

The use according to the first aspect of the present invention, wherein, the pharmaceutical composition further comprises a second therapeutic component, and the second therapeutic component is the active ingredient for antagonizing the muscle relaxant drug. In the examples, the second therapeutic component is neostigmine.

In the second aspect of the present invention, provided is an adamgammadex sodium preparation, comprising: a therapeutically effective amount of adamgammadex sodium, mannitol, and a balance of injection water.

The preparation according to the second aspect of the present invention comprises: 2-5 parts by weight of adamgammadex sodium, 0.1-1 parts by weight of mannitol, and 15-30 parts by weight of injection water.

The preparation according to the second aspect of the present invention comprises: 0.2-0.5 g of adamgammadex sodium per dose, 0.01-0.1 g of mannitol per dose, and a balance of injection water, and the volume of the preparation is 2 mL.

The preparation according to the second aspect of the present invention is used to reverse muscle relaxation induced by cisatracurium besylate.

In another preferred embodiment, the preparation is used to reverse residual neuromuscular blockade after muscle relaxation induced by cisatracurium.

In another preferred embodiment, the preparation is also used to improve adverse reactions during the antagonism of muscle relaxation induced by cisatracurium.

In a third aspect of the present invention, provided is an adamgammadex sodium preparation, wherein the preparation is a freeze-dried powder injection, and the preparation comprises: a therapeutically effective amount of adamgammadex sodium, sodium hydroxide or hydrochloric acid, and a balance of injection water.

In another preferred embodiment, the preparation comprises: 1-5 parts by weight of adamgammadex sodium, 15-30 parts by weight of injection water, and an appropriate amount of sodium hydroxide or hydrochloric acid.

In another preferred embodiment, the preparation comprises: 0.1-0.5 g of adamgammadex sodium per dose, an appropriate amount of sodium hydroxide or hydrochloric acid, and a balance of injection water, and the volume of the preparation is 2 mL.

In another preferred embodiment, the preparation is prepared by the following method: mixing a therapeutically effective amount of adamgammadex sodium with a prescribed dose of injection water, adjusting the pH to 6-9 using sodium hydroxide or hydrochloric acid, and then undergoing freeze-drying to obtain the freeze-dried powder injection preparation.

The preparation according to the third aspect of the present invention, wherein, the preparation is used to reverse

muscle relaxation induced by cisatracurium besylate.

In another preferred embodiment, the preparation is used to reverse residual neuromuscular blockade after muscle relaxation induced by cisatracurium .

In another preferred embodiment, the preparation is also use to improve the adverse reactions during the antagonism of the muscle relaxation induced by cisatracurium.

In a fourth aspect of the present invention, provided is a combination injectable preparation, wherein the preparation comprises: a therapeutically effective amount of adamgammadex sodium and neostigmine methylsulfate.

Preferably, the preparation further comprises: mannitol, a bacteriostat, a pH adjuster, a balance of injection water.

In another preferred embodiment, the bacteriostat is phenol.

In another preferred embodiment, the pH adjuster is sodium hydroxide or hydrochloric acid.

The combination injectable preparation according to the fourth aspect of the present invention, wherein, the preparation comprises: 2-5 parts by weight of adamgammadex sodium, 6-22 parts by weight of neostigmine methylsulfate, 0.1-1 weight part of mannitol, 0.01-0.1 weight part of a bacteriostat, an appropriate amount of pH adjuster, and a balance of injection water, and the volume of the preparation is 2 mL.

In another preferred embodiment, the pH of the preparation is 6-9.

## Advantageous Effects of the Invention

(1) The results of animal model experiments strongly demonstrate that adamgammadex sodium has the ability to antagonize the effects of depolarizing muscle relaxant cisatracurium. Specifically, at a dose of 10 mg/kg, adamgammadex sodium significantly shortened the residual muscle relaxation recovery time in a guinea pig muscle relaxation model induced by cisatracurium. As the dose of adamgammadex sodium increased, the 20 and 40 mg/kg dose groups showed a better effect in accelerating muscle relaxation recovery, exhibiting a certain dose-response relationship. The high-dose group of adamgammadex sodium (40 mg/kg), the positive control group (neostigmine 0.0625 mg/kg), and the combination group (adamgammadex sodium: 10 mg/kg; neostigmine: 0.03125 mg/kg) had the same residual muscle relaxation recovery effect in the guinea pig muscle relaxation model. The effects were all very significant, and there was no significant difference among the three groups, indicating that the antagonistic activity of adamgammadex sodium against cisatracurium is comparable to that of neostigmine.

(2) At the same time, it was also pleasantly discovered that, in terms of heart rate protection, the safety of adamgammadex sodium is significantly superior to that of neostigmine. Specifically, after administration of 0.0625 mg/kg neostigmine in the positive control group, heart rate decreased significantly, by as much as approximately 30%, posing a safety concern. In the combination group, the heart rate reduction was somewhat weakened after drug administration but did not fully recover. Administration of adamgammadex sodium at doses of 10, 20, and 40 mg/kg had no significant effect on blood pressure or heart rate in the guinea pig muscle relaxation model. Therefore, adamgammadex sodium has the potential to be developed into a clinical drug capable of antagonizing the depolarizing muscle relaxant cisatracurium.

(3) Adamgammadex sodium is indicated for antagonizing various degrees of neuromuscular blockade induced by steroidal non-depolarizing muscle relaxants such as rocuronium bromide during general anesthesia surgeries. It has currently completed Phase III clinical studies in China. If the application of antagonizing cisatracurium is successfully developed, it will further broaden its clinical application range, solve the muscarinic effects of neostigmine, and overcome the safety concerns associated with the combined use of atropine and neostigmine, namely: unstable heart rate performance, risk of tachycardia and arrhythmia, and postoperative cognitive disorders. The successful replacement of the neostigmine/atropine combination with adamgammadex sodium will benefit a broader population of patients undergoing general anesthesia surgeries.

It should be understood that, within the scope of the present invention, each of the above-mentioned technical features of the present invention and each of the technical features specifically described in the following (such as the examples) can be combined with each other to form new or preferred technical solutions. Due to space limitations, it will not be elaborated herein.

## DESCRIPTION OF THE DRAWINGS

Figure 1 shows muscle relaxation monitoring graphs of guinea pigs in each group. As shown in Figure 1, (A) shows the muscle relaxation monitoring graph of each of the 10 guinea pigs in the negative control group; (B) shows the muscle relaxation monitoring graph of each of the 10 guinea pigs in the positive control group; (C) shows the muscle relaxation monitoring graph of each of the 10 guinea pigs in the low-dose test group; (D) shows the muscle relaxation

monitoring graph of each of the 10 guinea pigs in the medium-dose test group; (E) shows the muscle relaxation monitoring graph of each of the 10 guinea pigs in the high-dose test group; (F) shows the muscle relaxation monitoring graph of each of the 10 guinea pigs in the combination group.

## DETAILED DESCRIPTION OF THE INVENTION

The present invention is further illustrated below in conjunction with specific examples. It should be understood that these examples are only for illustrating the invention and are not intended to limit the scope of the invention. The experimental methods in the following examples, for which specific conditions are not specified, are generally carried out under conventional conditions or conditions recommended by the manufacturer. Unless otherwise indicated, percentages and parts are calculated by weight.

### Example 1: Evaluation of Compound-Induced Sensitization Risk (Safety Screening 1)

1.Experimental Procedure

Wild-type AB strain zebrafish at 2 days post-fertilization (2 dpf) were randomly selected and placed in 24-well plates. Each group was with 6 replicate wells per group and 10 zebrafish per well. The following groups were set up: (1) Normal control group; (2) Positive control group (the used drug was mast cell sensitizer C48/80 at 1.5 μg/mL concentration); (3) Compound 1 (adamgammadex sodium) experimental group; (4) Compound 2 experimental group; (5) Compound 3 experimental group; (6) Compound 4 experimental group; (7) Compound 5 experimental group; (8) Compound 6 experimental group; (9) Compound 7 (sugammadex sodium) experimental group. In the experimental groups, Compound 1 was adamgammadex sodium, Compounds 2-6 were adamgammadex sodium analogues, and Compound 7 was sugammadex sodium. The volume of each well was 1 mL, and all experimental groups were treated with BAPNA (a specific detection reagent for mast cell degranulation) dissolved in water.

Table 1: Structural Formulas of Compounds 1-7

| Compound No. | Structure |
|---|---|
| 1 (Adamgammadex sodium) | 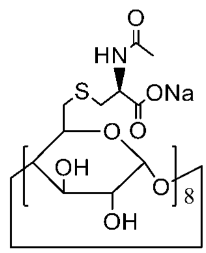 |
| 2 | 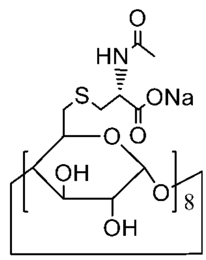 |
| 3 | 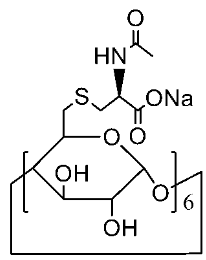 |

(continued)

| Compound No. | Structure |
|---|---|
| 4 | 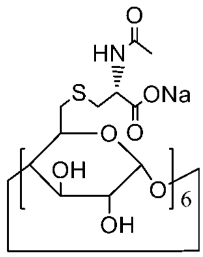 |
| 5 | 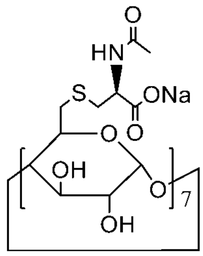 |
| 6 | 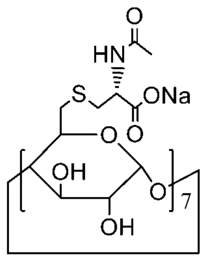 |
| 7(Sugammadex sodium) | 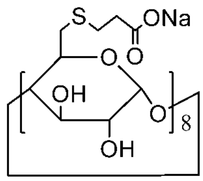 |

After 1 day of treatment at 28°C, the liquid was transferred to a 96-well plate, with 200 μL/well. The relative absorbance (OD value) of tryptase expression levels in each experimental group was measured using a microplate reader. The OD value statistical analysis results were used to evaluate the sensitization risk of the samples. Statistical analysis was performed using SPSS software, with $p < 0.05$ indicating a significant difference.

$$\text{Sensitization risk (\%)} = \frac{\text{OD(Sample group)} - \text{OD(Normal Control group)}}{\text{OD (Normal Control group)}} \times 100\%$$

2.Experimental Results

Table 2: Sensitization Risk of Each Compound

| Group | Dose (ng/tail) | OD value (mean ± SE) | Sensitization risk (%) |
|---|---|---|---|
| Normal Control | - | 0.054 ± 0.001 | - |
| Positive Control | - | 0.099 ± 0.003*** | 83 |
| Compound 1 Exp. Group | 500 | 0.060 ± 0.001* | 11 |
| Compound 2 Exp. Group | 500 | 0.052 ± 0.001 | -4 |
| Compound 3 Exp. Group | 500 | / | / |

(continued)

| Group | Dose (ng/tail) | OD value (mean ± SE) | Sensitization risk (%) |
|---|---|---|---|
| Compound 4 Exp. Group | 500 | / | / |
| Compound 5 Exp. Group | 500 | / | / |
| Compound 6 Exp. Group | 500 | / | / |
| Compound 7 Exp. Group | 500 | 0.079 ± 0.003*** | 46 |

Note: Data in the table are presented as mean ± standard error (mean ± SE).
*: $P < 0.05$; ***: $P < 0.001$ vs. Normal Control Group.

The results showed that the OD value of the positive control group was 0.099, significantly higher than that of the normal control group (0.054), indicating that the positive drug C48/80 has a high sensitization risk under the tested concentration conditions. The OD values of the Compound 1 (Adamgammadex sodium) Exp. group and the Compound 2 Exp. group were 0.060 and 0.052, respectively, similar to the normal control group (0.054), indicating almost no sensitization risk. The OD value of the Compound 7 (Sugammadex sodium) Exp. group was 0.079, which was somewhat increased compared to the normal control group (0.054), indicating a certain sensitization risk. In the Compounds 3, 4, 5, and 6 Exp. Group showed a higher rate of zebrafish death, so they were not evaluated.

3.Experimental Conclusion

Among the tested compounds, the sensitization risks of Compound 1 (Adamgammadex sodium), Compound 2, and Compound 7 (Sugammadex sodium) were significantly lower than that of the positive control. Among these, Compound 2 and Compound 1 (Adamgammadex sodium) showed no or almost no sensitization risk, making them suitable for subsequent drug development. Compound 7 (Sugammadex sodium) exhibited a low sensitization risk.

**Example 2: Effects of Adamgammadex sodium, Sugammadex Sodium, and Neostigmine on Immune Inflammation (Safety Screening 2)**

1.Experimental Procedure

An antibody array is a type of protein chip characterized by miniaturization, integration, and high throughput. It can be used to detect the expression abundance of proteins related to a specific physiological or pathological process. The principle involves arranging capture antibodies on a membrane or glass slide, incubating with the sample, adding biotin-labeled antibodies against the target proteins, and finally using HRP-streptavidin or fluorescein-streptavidin to detect the chip signal. Signals are detected using chemiluminescence or HiLyte™ Fluor 555-streptavidin.

In this experiment, ICR mice were randomly divided into a blank group, Adamgammadex sodium (1 g/kg) group, Sugammadex sodium (1 mg/kg) group, and Neostigmine (0.3 mg/kg) group, with 2 mice per group. Mice in each group given the corresponding dosage of compound via the tail vein once daily for 5 consecutive days. On the last day after administration, plasma from the 2 animals per group was mixed and detected using the Ary028 immune chip kit. Specific detection steps were as follows:

(1) Sample volume determination: 200 μL serum sample per Array unit. (2) Blocking protein chip: Each membrane was blocked with 2 mL of corresponding Array Buffer, incubated on a shaker for 1 hour. (3) Sample processing: Samples were diluted with corresponding Array Buffer to a volume of 1.5 mL per membrane, the corresponding Array unit was added, and incubated overnight with the Array protein membrane at 4°C. (4) Membranes were washed three times with corresponding Wash buffer, then diluted detection antibody was added and incubated at room temperature for 1 h. (5) Membranes were washed three times with corresponding Wash buffer, streptavidin-HRP was added and incubated for 30min at room temperature with shaking. (6) Membranes were washed three times with corresponding Wash buffer, 1 mL of chemiluminescent reagent was added to each well, and developed on a chemiluminescence imager. (7) Fluorescence intensity was used to represent the relative concentration of the corresponding protein.

2.Experimental Results

Table 3: Comparison of Fluorescence Intensity of Hypersensitivity-Related Factors

| Protein | Full Name of Protein | Blank | Adamgammadex sodium | Sugammadex sodium | Neostigmine |
|---|---|---|---|---|---|
| **CCL11** | Eosinophil activation chemokine | N.D. | N.D. | 2013.01 | 6286.81 |
| **CCL12** | Monocyte chemotactic receptor 5 | N.D. | 168.83 | 3096.64 | 5003.37 |
| **IL-1α** | Interleukin 1α | N.D. | N.D. | N.D. | 1891.15 |
| **IL-1β** | Interleukin 1β | N.D. | N.D. | N.D. | 1087.46 |
| **IL-3** | Interleukin 3 | N.D. | N.D. | N.D. | 1133.66 |
| **IL-4** | Interleukin 4 | N.D. | 225.32 | 1586.91 | 3939.66 |
| **IL-5** | Interleukin-5 | N.D. | N.D. | N.D. | 1864.09 |
| **IL-6** | Interleukin-6 | N.D. | 89.83 | 364.23 | 2321.39 |
| **IL-15** | Interleukin-15 | N.D. | N.D. | N.D. | 1503.8 |
| **GM-CSF** | Granulocyte-macrophage colony-stimulating factor | N.D. | N.D. | N.D. | 1679.67 |
| **TNF-α** | Tumor necrosis factor | N.D. | N.D. | 594.06 | 1116.77 |
| Note: "N.D." indicates that the serum concentration of the protein was below the minimum detection limit. | | | | | |

Table 4: Comparison of Fluorescence Intensity of Inflammation-Related Factors

| Protein | Full Name of Protein | Blank | Adamgammadex sodium | Sugammadex sodium | Neostigmine |
|---|---|---|---|---|---|
| **CCL17/TAR C** | Thymus activation regulatory chemokine | N.D. | N.D. | N.D. | 3653.32 |
| **CCL19/MIP 3β** | Macrophage inflammatory protein 3β | N.D. | N.D. | N.D. | 3385.33 |
| **CCL20/MIP 3α** | Macrophage inflammatory protein 3α | N.D. | N.D. | N.D. | 4440.55 |
| **CCL21/6Cki ne** | Lymphocyte chemokine CCL21 | N.D. | 8082.61 | 27027.2 | 17119.34 |
| **CCL22/MD C** | Macrophage-derived chemokine | N.D. | 738.38 | 2578.89 | 4762.19 |
| **CXCL1/KC** | Neutrophil chemokine | N.D. | N.D. | N.D. | 2935.82 |
| **CXCL9/MIG** | IFN-γ-induced monocyte factor | N.D. | N.D. | 3617.92 | 1730.04 |
| **CXCL10/IP-10** | Interferon-induced protein 10 | N.D. | N.D. | N.D. | 1971.99 |
| **CXCL11/I-TAC** | Interferon-induced T cell chemokines | N.D. | N.D. | N.D. | 2397.51 |
| **CXCL13** | CXCL13 (CXC family chemokine) | N.D. | N.D. | N.D. | 3607.1 |
| **CXCL16** | CXCL16 (CXC family chemokine) | N.D. | N.D. | N.D. | 2426.598 |
| **CX3CL1/ Gractalkine** | Neural chemokine | N.D. | N.D. | N.D. | 2935.07 |
| **CD160** | Leukocyte differentiation antigen160 | N.D. | N.D. | 187.67 | 4298.1 |
| **IL-2** | Interleukin-2 | N.D. | N.D. | N.D. | 1107.85 |
| **IL-7** | Interleukin-7 | N.D. | 1850.57 | 1922.84 | 3226.41 |
| **IL-10** | Interleukin-10 | N.D. | N.D. | N.D. | 1601.99 |
| **IL-11** | Interleukin-11 | N.D. | N.D. | 216.49 | 2195.99 |

(continued)

| Protein | Full Name of Protein | Blank | Adamgammadex sodium | Sugammadex sodium | Neostigmine |
|---|---|---|---|---|---|
| **IL-12p40** | Interleukin IL-12p40 | N.D. | N.D. | 1049.34 | 2936.85 |
| **IL-13** | Interleukin-13 | N.D. | N.D. | N.D. | 1609.58 |
| **IL-15** | Interleukin -15 | N.D. | N.D. | N.D. | 1503.8 |
| **IL-17A** | Interleukin -17A | N.D. | N.D. | N.D. | 783.45 |
| **IL-22** | Interleukin-22 | N.D. | N.D. | N.D. | 1253.05 |
| **IL-23** | Interleukin-23 | N.D. | N.D. | N.D. | 1483.72 |
| **IL-27p28** | Interleukin-27p28 | N.D. | N.D. | N.D. | 1575.98 |
| **IL-28** | Interleukin-28 | N.D. | 244.77 | 2440.68 | 3505.78 |
| **IL-33** | Interleukin-33 | N.D. | N.D. | 592.77 | 1897.86 |
| **Chemerin** | Chemotactic factor | N.D. | 2680.26 | 4629.24 | 7955.57 |
| **C-Reactive Protein/CRP** | C-reactive protein | N.D. | 5549.81 | 8550.52 | 11999.12 |
| **M-CSF** | Macrophage colony-stimulating factor | N.D. | 871.82 | 5871.05 | 6478.33 |
| **Myeloperoxidase** | Mastoperoxidase | 579.7 5 | 1927.54 | 4645.21 | 6395.44 |
| **Pentraxin 3** | Pertussis toxin 3 | N.D. | N.D. | 4555.53 | 3694.57 |
| **TIM-1/KIM-1/ HAVCR** | Hepatitis A virus receptor-1 (inflammation/ immunity) | N.D. | N.D. | 1023.1 | 1197.17 |
| **BAFF/BlyS/ TNFSF13B** | B cell activation factor | N.D. | 6659.29 | 13375.34 | 12170.94 |
| **G-CSF** | Granulocyte colony-stimulating factor | N.D. | N.D. | N.D. | 1289.56 |
| **M-CSF** | Macrophage lysis-stimulating factor | N.D. | 871.82 | 5871.05 | 6478.33 |
| **Pentraxin** 3/TSG-14 | Pertussis toxin 3 | N.D. | N.D. | 4555.53 | 3694.57 |

Note: "N.D." indicates that the serum concentration of the protein was below the minimum detection limit.

A total of 11 hypersensitivity-related factors and 45 inflammation-related factors were detected. As shown in Tables 3 and 4, the detected levels of 11 hypersensitivity-related factors and 37 inflammation-related factors in serum were all below the minimum detection limit in the blank group. Compared with the Sugammadex sodium group and the neostigmine group, the detected levels of all hypersensitivity and inflammation-related factors in the adamgammadex sodium group were the lowest or were below the detection limit. This indicates that adamgammadex sodium has the smallest impact on inducing immune inflammation in mice, followed by Sugammadex sodium, with neostigmine having the most severe impact.

3.Experimental Conclusion

The analysis of the effects of the three muscle relaxant antagonists (adamgammadex sodium, Sugammadex sodium, and neostigmine) on immune inflammation in mice in this experiment showed that adamgammadex sodium had the smallest impact on inducing immune inflammation. Many hypersensitivity reactions and inflammation-related factors were not induced, falling below the minimum detection limit, indicating that this drug has excellent safety in terms of immune inflammation. Sugammadex sodium had a second-lowest impact on inducing immune inflammation in mice, suggesting that the safety of this drug in terms of immune inflammation is average. Neostigmine had the most severe impact on inducing immune inflammation in mice, leading to increased secretion of the vast majority of hypersensitivity reactions and inflammation-related factors, indicating that the safety of this drug in terms of immune inflammation has significant risks.

**Example 3: Effect of adamgammadex sodium on antagonism of residual muscle relaxant after administration of muscle relaxant cisatracurium in anesthetized guinea pigs (efficacy experiment)**

1.Experimental Procedure

Ninety (90) qualified Hartley guinea pigs, male, were used. Sixty (60) animals were randomly divided into 6 experimental groups based on body weight using a stratified random method: negative control group, positive control group, low-dose test group, medium-dose test group, high-dose test group, and combination group, with 10 animals per group (n=10). The remaining animals served as spares. The negative control group received 0.9% sodium chloride. The positive control group received 0.0625 mg/kg neostigmine methylsulfate (referred to as neostigmine). The low-dose test group received 10 mg/kg adamgammadex sodium. The medium-dose test group received 20 mg/kg adamgammadex sodium. The high-dose test group received 40 mg/kg adamgammadex sodium. The combination group received 10 mg/kg adamgammadex sodium and 0.03125 mg/kg neostigmine. The administration volume for all groups was 1 mL/kg.

Adaptive feeding was conducted after the quarantine period. On the day of the experiment, guinea pigs were weighed, administered atropine (0.1 mg/kg) intramuscularly, and then anesthetized with an intraperitoneal injection of anesthetic (pentobarbital sodium 10 mg/kg, urethane 1000 mg/kg). After fixation, the animals were intubated and connected to a small animal ventilator. Both external jugular veins and the right carotid artery were cannulated. The left jugular vein was used for cisatracurium infusion, the right jugular vein for injection of different test substances, and the carotid artery was connected to a multi-channel physiological signal acquisition and processing system for continuous monitoring of arterial blood pressure and heart rate. Electrode patches were subcutaneously placed behind the left femur and tibia of the guinea pig, and two stimulating electrodes of the neuromuscular monitor were fixed to the two electrode patches. The left hind paw was fixed on a custom-made platform. A sensor was fixed to the skin surface over the gastrocnemius muscle of the left hind limb, and a skin temperature probe was fixed to a flat area of skin. Continuous single-twitch stimulation was applied at 1 Hz. After the twitch height stabilized, the mode was switched to train-of-four stimulation (TOF mode, stimulus current 5 mA, frequency 2 Hz, pulse width 0.2 ms, interval between trains 15 s) for at least 5 min. Calibration was then performed using Calibration Mode 2, followed by TOF mode stimulation. After obtaining a stable stimulation response for at least 2 min, cisatracurium solution was infused intravenously using a dual-channel micro-infusion pump, and the start time of infusion was recorded. The muscle relaxation model was considered successful when continuous infusion of cisatracurium resulted in the disappearance of T4/T1 (TOFr) and T1 was below 15%. At this point, cisatracurium infusion was stopped. After stopping the infusion, neuromuscular function was allowed to recover spontaneously. When neuromuscular function reached moderate recovery, the test drug formulation was administered intravenously. Neuromuscular function was allowed to recover completely, and the administration time was recorded (Figure 1). Using the time of spontaneous recovery to moderate neuromuscular function (TOF50) as zero point, the times for recovery from TOF50 to TOF75, TOF75 to TOF90, and TOF50 to TOF90 were calculated for each animal (Table 1-2). Arterial blood pressure (systolic, diastolic, mean arterial pressure) and heart rate were recorded before cisatracurium infusion, at the time cisatracurium infusion was stopped, at the time of moderate neuromuscular recovery, and at the time of complete neuromuscular function recovery (Tables 3-7). Data were expressed as Mean ± SD. Statistical T-tests were performed using SPSS software to evaluate differences in blood pressure, heart rate, and residual neuromuscular blockade recovery times. $P < 0.05$ was considered statistically significant.

2.Experimental Results

(1) The effect of adamgammadex sodium on the residual muscle relaxation recovery time in the muscle relaxation model animals

Table 5 The effect of adamgammadex sodium antagonizing cisatracurium on residual muscle relaxation recovery time in the muscle relaxation model animals

| | | Residual Muscle Relaxation Recovery Time (seconds) | | | |
|---|---|---|---|---|---|
| **Group** | **Test Comp.** | **tTOF0-50** | **tTOF50-75** | **tTOF75-90** | **tTOF50-90** |
| Negative Control Group | 0.9%NaCl | 1191±343 | 225±97 | 230±145 | 455±201 |
| Positive Control Group | Neostigmine | 1134±281 | 101±71** | 87±68* | 188±86** |
| Low-Dose Test Group | Adamgammadex sodium | 1093±427 | 143±85 | 105±78* | 248±139* |
| Medium-Dose Test Group | Adamgammadex sodium | 1349±381 | 159±72 | 120±99 | 279±123* |

(continued)

| Group | Test Comp. | Residual Muscle Relaxation Recovery Time (seconds) | | | |
|---|---|---|---|---|---|
| | | tTOF0-50 | tTOF50-75 | tTOF75-90 | tTOF50-90 |
| High-Dose Test Group | Adamgammadex sodium | 1413±693 | 131±144 | 71±52** | 201±131** |
| Combination Group | Adamgammadex sodium Neostigmine | 1404±736 | 90±52** | 84±71* | 174±108** |

Note: Data in the table are presented as mean ± standard error (mean ± SE).
*: P < 0.05; ***: P < 0.001 vs. Negative Control Group. 10 guinea pigs per group.

The results showed that compared with the negative control group, there were no significant differences in tTOF0-50 values among the groups (P > 0.05), indicating that the spontaneous recovery of neuromuscular function before administration of the test substances was generally consistent. After the neuromuscular function spontaneously recovered to a moderate level, the test substance administration formulation was administered, and the neuromuscular function fully recovered. The residual muscle relaxation recovery times tTOF50-75, tTOF75-90, and tTOF50-90 were significantly shortened in the positive control group (P < 0.05-0.01). The residual muscle relaxation recovery times tTOF75-90 and tTOF50-90 were significantly shortened in the low-dose test group (P < 0.05). The residual muscle relaxation recovery time tTOF50-90 was significantly shortened in the medium-dose test group (P < 0.05). The residual muscle relaxation recovery times tTOF75-90 and tTOF50-90 were significantly shortened in the high-dose test group (P < 0.01). The residual muscle relaxation recovery times tTOF50-75, tTOF75-90, and tTOF50-90 were significantly shortened in the combination group (P < 0.05-0.01). These results indicate that, under the conditions of this experiment, different doses of adamgammadex sodium could significantly shorten the residual muscle relaxation recovery time in the cisatracurium-induced guinea pig muscle relaxation model. The tTOF50-90 values for the negative control, positive control, low-, medium-, high-dose test, and combination groups were (455 ± 201) s, (188 ± 86) s, (248 ± 139) s, (279 ± 123) s, (201 ± 131) s, and (174 ± 108) s, respectively. After administration of the drugs in the positive control, low-, medium-, high-dose test, and combination groups, tTOF50-90 was shortened by 58.7%, 45.5%, 38.7%, 55.8%, and 61.8%, respectively (Degree of tTOF50-90 shortening = (tTOF50-90 value of negative control group - tTOF50-90 value of experimental group) / tTOF50-90 value of negative control group). This indicates that low, medium, and high doses of adamgammadex sodium have a significant effect on residual muscle relaxation recovery in the cisatracurium-induced guinea pig muscle relaxation model, and the effect is comparable to that of neostigmine administration or the combination of adamgammadex sodium and neostigmine.

(2) Effect of adamgammadex sodium on residual muscle relaxation recovery time ratios in the muscle relaxation model animals

Considering the potential differences in the spontaneous muscle relaxation recovery time tTOF0-50 among individual guinea pigs before test compounds administration, the ratios of residual muscle relaxation recovery times tTOF50-75, tTOF75-90, and tTOF50-90 to the muscle relaxation recovery time tTOF0-50 (RtTOF50-75/0-50, RtTOF75-90/0-50, and RtTOF50-90/0-50) were used as outcome measures.

Table 6: Effect of adamgammadex sodium antagonizing cisatracurium on residual muscle relaxation recovery time ratios in muscle relaxation model animals

| Group | Test Comp. | Residual Muscle Relaxation Recovery Time Ratios | | |
|---|---|---|---|---|
| | | RtTOF50-75/0-50 | RtTOF75-90/0-50 | RtTOF50-90/0-50 |
| Negative Control Group | 0.9%NaCl | 0.22±0.14 | 0.20±0.13 | 0.42±0.22 |
| Positive Control Group | Neostigmine | 0.09±0.07* | 0.09±0.07* | 0.18±0.09** |
| Low-Dose Test Group | Adamgammadex sodium | 0.14±0.10 | 0.09±0.05* | 0.23±0.13* |
| Medium-Dose Test Group | Adamgammadex sodium | 0.12±0.05 | 0.09±0.07* | 0.22±0.08* |
| High-Dose Test Group | Adamgammadex sodium | 0.10±0.11 | 0.06±0.06** | 0.16±0.11** |

(continued)

| Group | Test Comp. | Residual Muscle Relaxation Recovery Time Ratios | | |
|---|---|---|---|---|
| | | RtTOF50-75/0-50 | RtTOF75-90/0-50 | RtTOF50-90/0-50 |
| Combination Group | Adamgammadex sodium Neostigmine | 0.09±0.08* | 0.09±0.09* | 0.17±0.17* |
| Note: Data in the table are presented as mean ± standard error (mean ± SE). *: P < 0.05; ***: P < 0.001 vs. Negative Control Group. 10 guinea pigs per group. | | | | |

The results showed that compared with the negative control group, the residual muscle relaxation recovery time ratios RtTOF50-75/0-50, RtTOF75-90/0-50, and RtTOF50-90/0-50 were significantly decreased in the positive control group (P < 0.05-0.01). The residual muscle relaxation recovery time ratios RtTOF75-90/0-50 and RtTOF50-90/0-50 were significantly decreased in the low- and medium-dose test groups (P < 0.05). The residual muscle relaxation recovery time ratios RtTOF75-90/0-50 and RtTOF50-90/0-50 were significantly decreased in the high-dose test group (P < 0.01). The residual muscle relaxation recovery time ratios RtTOF50-75/0-50, RtTOF75-90/0-50, and RtTOF50-90/0-50 were significantly decreased in the combination group (P < 0.05). These results indicate that, under the conditions of this experiment, different doses of adamgammadex sodium could significantly accelerate residual muscle relaxation recovery in the cisatracurium-induced guinea pig muscle relaxation model, exhibiting a certain dose-response relationship. Furthermore, compared with the positive control group, there were no significant differences in the residual muscle relaxation recovery time ratios RtTOF50-75/0-50, RtTOF75-90/0-50, and RtTOF50-90/0-50 for the low-, medium-, and high-dose test groups (P > 0.05), indicating that the combination group and each test dose group had similar efficacy in promoting residual muscle relaxation recovery in the model animals as the positive control group. The RtTOF50-90/0-50 values for the negative control, positive control, low-, medium-, high-dose test, and combination groups were (0.42 ± 0.22), (0.18 ± 0.09), (0.23 ± 0.13), (0.22 ± 0.08), (0.16 ± 0.11), and (0.17 ± 0.17), respectively. The results show that RtTOF50-90/0-50 was significantly decreased in all administration groups, and the effect on residual muscle relaxation recovery was essentially consistent among the combination group, each test dose group, and the positive control group.

(3) Effect of Adamgammadex sodium on blood pressure in the muscle relaxation model animals

Table 7: Effect of adamgammadex sodium antagonizing cisatracurium on mean systolic pressure in muscle relaxation model animals

| Group | Test Comp. | Dose (mg/kg) | Mean Systolic Pressure mSP(mmHg) | | | | |
|---|---|---|---|---|---|---|---|
| | | | Muscle Relaxation Start | Muscle Relaxation End | Administration | Moderate Recovery | Complete Recovery |
| Negative Control Group | 0.9%NaCl | 0 | 74±14 | 77±15 | 81±15 | 83±18 | 84±17 |
| Positive Control Group | Neostigmine | 0.0625 | 71±12 | 71±10 | 76±15 | 79±13 | 78±13 |
| Low-Dose Test Group | Adamgammadex sodium | 10 | 79±18 | 79±12 | 85±18 | 83±12 | 86±16 |
| Medium-Dose Test Group | Adamgammadex sodium | 20 | 74±9 | 75±10 | 80±8 | 84±7 | 84±7 |
| High-Dose Test Group | Adamgammadex sodium | 40 | 74±12 | 75±11 | 81±10 | 83±9 | 84±11 |
| Combination Group | Adamgammadex sodium<br>Neostigmine | 10<br>0.03125 | 71±10 | 74±10 | 74±7 | 80±11 | 79±10 |
| Note: Data in the table are presented as mean ± standard error (mean ± SE). No significant differences were observed between the groups, P > 0.05 vs. Negative Control Group. 10 guinea pigs per group. | | | | | | | |

Table 8: Effect of adamgammadex sodium antagonizing cisatracurium on mean diastolic pressure in muscle relaxation model animals

| Group | Test Comp. | Dose (mg/kg) | Mean Diastolic Pressure mDP(mmHg) | | | | |
|---|---|---|---|---|---|---|---|
| | | | Muscle Relaxation Start | Muscle Relaxation End | Administration | Moderate Recovery | Complete Recovery |
| Negative Control Group | 0.9%NaCl | 0 | 57+12 | 61+14 | 63+13 | 65+16 | 65+14 |
| Positive Control Group | Neostigmine | 0.0625 | 56±9 | 57+6 | 60+10 | 61+12 | 58+15 |
| Low-Dose Test Group | Adamgammadex sodium | 10 | 61+14 | 62+9 | 67+13 | 65+8 | 68+11 |
| Medium-Dose Test Group | Adamgammadex sodium | 20 | 56+8 | 57+9 | 61+8 | 64+6 | 64+6 |
| High-Dose Test Group | Adamgammadex sodium | 40 | 56+12 | 56+9 | 60+7 | 62±8 | 63±9 |
| Combination Group | Adamgammadex sodium<br>Neostigmine | 10<br>0.03125 | 54+9 | 57±8 | 57+4 | 61+12 | 58+6 |

Note: Data in the table are presented as mean ± standard error (mean ± SE). No significant differences were observed between the groups, P > 0.05 vs. Negative Control Group. 10 guinea pigs per group.

Table 9: Effect of adamgammadex sodium antagonizing cisatracurium on mean arterial pressure in muscle relaxation model animals

| Group | Test Comp. | Dose (mg/kg) | Mean Arterial Pressure mAP(mmHg) | | | | |
|---|---|---|---|---|---|---|---|
| | | | Muscle Relaxation Start | Muscle Relaxation End | Administration | Moderate Recovery | Complete Recovery |
| Negative Control Group | 0.9%NaCl | 0 | 65+13 | 68+14 | 72+14 | 73+17 | 74+15 |
| Positive Control Group | Neostigmine | 0.0625 | 62+11 | 63+8 | 68+12 | 69+12 | 67+15 |
| Low-Dose Test Group | Adamgammadex sodium | 10 | 70±16 | 70+11 | 75+15 | 74+10 | 76+13 |
| Medium-Dose Test Group | Adamgammadex sodium | 20 | 64±8 | 65+10 | 70+8 | 74+7 | 73+6 |
| High-Dose Test Group | Adamgammadex<br>sodium | 40 | 64+12 | 65+10 | 70±8 | 72+8 | 73+10 |
| Combination Group | Adamgammadex sodium<br>Neostigmine | 10<br>0.03125 | 61±9 | 65+9 | 65+5 | 70+12 | 68±8 |

Note: Data in the table are presented as mean ± standard error mean ± SE). No significant differences were observed between the groups, P > 0.05 vs. Negative Control Group. 10 guinea pigs per group.

Table 10: Effect of adamgammadex sodium antagonizing cisatracurium on mean pulse pressure in muscle relaxation model animals

| Group | Test Comp. | Dose (mg/kg) | Mean Pulse Pressure (mmHg) | | | | |
|---|---|---|---|---|---|---|---|
| | | | Muscle Relaxation Start | Muscle Relaxation End | Administration | Moderate Recovery | Complete Recovery |
| Negative Control Group | 0.9%NaCl | 0 | 17±2 | 16+3 | 18+6 | 18+6 | 19+6 |
| Positive Control Group | Neostigmine | 0.0625 | 15+4 | 14±5 | 17±8 | 19±8 | 19+6 |
| Low-Dose Test Group | Adamgammadex sodium | 10 | 18±5 | 17+4 | 19+6 | 18±5 | 18+6 |
| Medium-Dose Test Group | Adamgammadex sodium | 20 | 18±3 | 18±3 | 19±3 | 20+3 | 20+4 |
| High-Dose Test Group | Adamgammadex sodium | 40 | 18+4 | 19±5 | 21±5 | 21+4 | 21+5 |
| Combination Group | Adamgammadex sodium<br>Neostigmine | 10<br>0.03125 | 17±5 | 17±5 | 18±5 | 20+6 | 21+5 |

Note: Data in the table are presented as mean ± standard error (mean ± SE). No significant differences were observed between the groups, P > 0.05 vs. Negative Control Group. 10 guinea pigs per group.

The results showed that compared with the negative control group animals, there were no significant differences in mean systolic pressure, mean diastolic pressure, mean arterial pressure, and mean pulse pressure in the positive control group, low-, medium-, high-dose test groups, and combination group animals before cisatracurium infusion, at the time of stopping cisatracurium infusion, at the time of moderate neuromuscular recovery, and at the time of complete neuromuscular function recovery (P > 0.05).

(4) Effect of Adamgammadex sodium on heart rate in the muscle relaxation model animals

Table 11: Effect of adamgammadex sodium antagonizing cisatracurium on heart rate in muscle relaxation model animals

| Group | Test Comp. | Dose (mg/kg) | Heart rate (bmp) | | | | |
|---|---|---|---|---|---|---|---|
| | | | Muscle Relaxation Start | Muscle Relaxation End | Administration | Moderate Recovery | Complete Recovery |
| Negative Control Group | 0.9%NaCl | 0 | 247+37 | 245±31 | 259+17 | 263+16 | 265±16 |
| Positive Control Group | Neostigmine | 0.0625 | 240+14 | 239+11 | 238+42 | 181±54** | 157±49*** |
| Low-Dose Test Group | Adamgammadex sodium | 10 | 259+30 | 261+20 | 269+20 | 268+20 | 270+18 |
| Medium-Dose Test Group | Adamgammadex sodium | 20 | 242+43 | 247+42 | 259+38 | 261+35 | 263+36 |
| High-Dose Test Group | Adamgammadex sodium | 40 | 243+27 | 249±21 | 258+18 | 257+18 | 258+16 |

(continued)

| Group | Test Comp. | Dose (mg/kg) | Heart rate (bmp) | | | | |
|---|---|---|---|---|---|---|---|
| | | | Muscle Relaxation Start | Muscle Relaxation End | Administration | Moderate Recovery | Complete Recovery |
| Combination Group | Adamgammadex sodium<br>Neostigmine | 10<br>0.03125 | 239+19 | 244±22 | 247+22 | 219±41** | 190±61** |

Note: Data in the table are presented as mean ± standard error (mean ± SE).
**: P < 0.01; ***: P < 0.001 vs. Negative Control Group. 10 guinea pigs per group.

The results showed that compared with the negative control group animals, there were no significant differences in heart rate in the low-, medium-, and high-dose test group animals before cisatracurium infusion, at the time of stopping cisatracurium infusion, at the time of moderate neuromuscular recovery, and at the time of complete neuromuscular function recovery (P > 0.05). This indicates that low, medium, and high doses of adamgammadex sodium test substance have no significant effect on heart rate at the time of moderate and complete neuromuscular recovery, and the drug exhibits good safety regarding its effect on heart rate.

Compared with the negative control group animals, there were also no significant differences in heart rate in the positive control group and combination group animals before cisatracurium infusion and at the time of stopping cisatracurium infusion (P > 0.05). However, compared with the negative control group animals, heart rate in the positive control group showed significant differences at the time of moderate neuromuscular recovery and complete neuromuscular function recovery (P < 0.01 or 0.001), with a decrease of 35% at complete muscle relaxation recovery (Degree of heart rate decrease at complete muscle relaxation recovery = (heart rate at start of muscle relaxation - heart rate at complete recovery) / heart rate at start of muscle relaxation). This indicates a significant side effect of neostigmine on heart rate. In the combination group animals, heart rate showed significant differences at the time of moderate neuromuscular recovery and complete neuromuscular function recovery (P < 0.01), with a decrease of 21% at complete muscle relaxation recovery (Degree of heart rate decrease at complete muscle relaxation recovery = (heart rate at start of muscle relaxation - heart rate at complete recovery) / heart rate at start of muscle relaxation), indicating that the combination of adamgammadex sodium and neostigmine mitigated the heart rate reduction effect but did not fully restore it.

3.Experimental Conclusion

Under the conditions of this experiment, adamgammadex sodium at a dose of 10 mg/kg significantly shortened the residual muscle relaxation recovery time in the cisatracurium-induced guinea pig muscle relaxation model, with an effect comparable to that of 0.0625 mg/kg neostigmine. Administration of 10, 20, and 40 mg/kg of adamgammadex sodium to guinea pigs in the cisatracurium muscle relaxation model showed no effect on animal blood pressure or heart rate, whereas administration of 0.0625 mg/kg neostigmine had a significant side effect of lowering heart rate. This indicates that adamgammadex sodium is significantly superior to neostigmine in terms of safety concerning heart rate protection.

As mentioned in the background of the specification, sugammadex sodium does not bind to atracurium or cisatracurium; conversely, after sugammadex sodium administration, the onset of cisatracurium-induced neuromuscular blockade is faster and the depth of blockade is greater. As early as 2006, H. D. de Boer et al. also found that sugammadex sodium was ineffective in reversing atracurium-induced neuromuscular blockade. Considering its clinical side effects such as hypersensitivity, allergic reactions, and QT prolongation, sugammadex sodium was not included as a control group in this experiment.

Given that cisatracurium holds more than 70% of the domestic muscle relaxant market share and also accounts for a high share internationally, its only antagonist is neostigmine, which has significant limitations and side effects that cannot meet clinical needs. The adamgammadex sodium of the present invention is non-inferior to neostigmine in efficacy, while not exhibiting the adverse reactions associated with neostigmine. As a new-generation product in this field, it can meet clinical needs and fill a market gap, which is of great significance.

**Example 4: Preparation of an injectable solution**

Table 12: Formulation Table

| Material | Prescription dosage (1000 unit) | Function |
|---|---|---|
| **Adamgammadex Sodium** | 0.2-0.5kg | Active Ingredient |
| **Mannitol** | 0.01 -0.1kg | Tonicity Adjuster |
| **Injection Water** | q.s. | Solvent |
| **Total Volume(mL)** | 2000 | -- |

According to the prescription dosage, mannitol was weighed and added to approximately 80% of the total volume of injection water, and stirred until dissolved. Then, the prescription dosage of adamgammadex sodium was added, stirred until dissolved, and water for injection was added to make up the total volume. The solution was sterilized by filtration through a 0.2 μm microporous membrane, dispensed into medium borosilicate glass ampoules, 2 mL per ampoule, purged with nitrogen, sealed, and sterilized at 121°C for 15 minutes to obtain the adamgammadex sodium injectable solution.

**Example 5: Preparation of a combination injectable solution**

Table 13: Formulation Table

| Material | Prescription dosage (1000 unit) | Function |
|---|---|---|
| **Adamgammadex Sodium** | 0.2-0.5kg | Active Ingredient |
| **Neostigmine Methylsulfate** | 0.625-2.17g | Active Ingredient |
| **Mannitol** | 0.01 -0.1kg | Tonicity Adjuster |
| **Phenol** | 1-10g | Bacteriostat |
| **Sodium Acetate** | 0.1-0.5g | pH Adjuster |
| **Acetate** | q.s. | pH Adjuster |
| **Sodium Hydroxide** | q.s. | pH Adjuster |
| **Injection Water** | q.s. | Solvent |
| **Total Volume(mL)** | 2000 | -- |

According to the prescription dosage, mannitol and sodium acetate was weighed and added to approximately 80% of the total volume of injection water, and stirred until dissolved. Then, the prescription dosage of adamgammadex sodium and neostigmine methylsulfate was added, stirred until dissolved. Then, the prescription dose of phenol was added and stirred until dissolved. An appropriate amount of acetic acid solution or sodium hydroxide solution was used to adjust the pH to 5.5 ± 0.5. Injection water was added to make up the total volume. The solution was sterilized by filtration through a 0.2 μm microporous membrane, dispensed into medium borosilicate glass ampoules, 2 mL per ampoule, purged with nitrogen, sealed, and sterilized at 121°C for 15 minutes to obtain the adamgammadex sodium and neostigmine methylsulfate combination injectable solution.

**Example 6: Preparation of a freeze-dried powder injection**

Table 14: Formulation Table

| Material | Prescription dosage | Function |
|---|---|---|
| **Adamgammadex Sodium** | 0.1-0.5g | Active Ingredient |
| **Sodium Hydroxide or Hydrochloric Acid** | q.s. | pH Adjuster |
| **Injection Water** | To 2mL | Solvent |

The prescription dosage of adamgammadex sodium was added to 80% of the prescription dosage of injection water and stirred to dissolve. Injection water was added to reach the prescription dosage. Activated carbon (0.1 - 0.5%) was added to the solution and stirred for several tens of minutes. The activated carbon was removed by filtration for

decolorization. The pH of the solution was adjusted to 6-9 using sodium hydroxide solution or hydrochloric acid solution. Samples were taken for semi-finished product testing. The solution was filtered again through a 0.22 μm filter and filled into vials. Freeze-dried, stoppering, capping, and packaging were performed according to the set lyophilization parameters.

All references mentioned in the present invention are cited as references in this application, as if each reference were cited separately. In addition, it should be understood that after reading the above teaching content of the present invention, those skilled in the art can make various changes or modifications to the present invention, and these equivalent forms also fall within the scope of the claims attached to this application.

## Claims

1. A use of adamgammadex sodium in the preparation of a pharmaceutical composition for reversing muscle relaxation induced by a benzylisoquinoline muscle relaxant.

2. The use according to claim 1, wherein, the pharmaceutical composition is further used to reverse residual neuromuscular blockade after muscle relaxation induced by a benzylisoquinoline muscle relaxant;
In another preferred embodiment, the benzylisoquinoline muscle relaxant is cisatracurium.

3. The use according to claim 1, wherein, the pharmaceutical composition is further used to alleviate the adverse reactions during the antagonism of muscle relaxation induced by cisatracurium.

4. The use according to claim 3, wherein, the adverse reactions are selected from the group consisting of: decreased heart rate, decreased blood pressure, and gastrointestinal discomfort.

5. The use according to claim 1, wherein, the pharmaceutical composition further comprises a second therapeutic component, and the second therapeutic component is the active ingredient for antagonizing the muscle relaxant drug.

6. The use according to claim 5, wherein, the second therapeutic component is neostigmine.

7. An adamgammadex sodium preparation, wherein, the preparation comprises: a therapeutically effective amount of adamgammadex sodium, mannitol, a balance of injection water;

   In another preferred embodiment, the preparation comprises: 2-5 parts by weight of adamgammadex sodium, 0.1-1 parts by weight of mannitol, and 15-30 parts by weight of injection water;
   In another preferred embodiment, the preparation comprises: 0.2-0.5 g of adamgammadex sodium per dose, 0.01-0.1 g of mannitol per dose, and the volume of the preparation is 2 mL.

8. The preparation according to claim 7, wherein, the preparation is used to reverse muscle relaxation induced by cisatracurium besylate;

   In another preferred embodiment, the preparation is used to reverse residual neuromuscular blockade after muscle relaxation induced by cisatracurium;
   In another preferred embodiment, the preparation is also used to alleviate adverse reactions during the antagonism of muscle relaxation induced by cisatracurium.

9. An adamgammadex sodium preparation, wherein, the preparation is a freeze-dried powder injection, and the preparation comprises: a therapeutically effective amount of adamgammadex sodium, a pH adjuster, and a balance of injection water;

   In another preferred embodiment, the preparation comprises: 1-5 parts by weight of adamgammadex sodium, 15-30 parts by weight of injection water, and an appropriate amount of a pH adjuster; preferably, the pH adjuster is sodium hydroxide or hydrochloric acid;
   In another preferred embodiment, the preparation comprises: 0.1-0.5 g of adamgammadex sodium per dose, an appropriate amount of sodium hydroxide or hydrochloric acid, and a balance of injection water, and the volume of the preparation is 2 mL;
   In another preferred embodiment, the preparation is prepared by a method comprising: mixing a therapeutically effective amount of adamgammadex sodium with a prescribed dose of injection water, adjusting the pH to 6-9 using sodium hydroxide or hydrochloric acid, and then undergoing freeze-drying to obtain the freeze-dried

powder injection preparation.

10. The preparation according to claim 9, wherein, the preparation is used to reverse muscle relaxation induced by cisatracurium;

    In another preferred embodiment, the preparation is used to reverse residual neuromuscular blockade after muscle relaxation induced by cisatracurium;
    In another preferred embodiment, the preparation is also use to alleviate adverse reactions during the antagonism of the muscle relaxation induced by cisatracurium.

11. A combination injectable preparation, wherein, the preparation comprises: a therapeutically effective amount of adamgammadex sodium and neostigmine methylsulfate;

    Preferably, the preparation further comprises: mannitol, a bacteriostat, a pH adjuster, and a balance of injection water;
    In another preferred embodiment, the bacteriostat is phenol;
    In another preferred embodiment, the pH adjuster is sodium hydroxide or hydrochloric acid.

12. The combination injectable preparation according to claim 11, wherein, the preparation comprises: 2-5 parts by weight of adamgammadex sodium, 6-22 parts by weight of neostigmine methylsulfate, 0.1-1 parts by weight of mannitol, 0.01-0.1 parts by weight of bacteriostat, an appropriate amount of pH adjuster, and a balance of injection water, and the volume of the preparation is 2 mL;
    In another preferred embodiment, the pH of the preparation is 6-9.

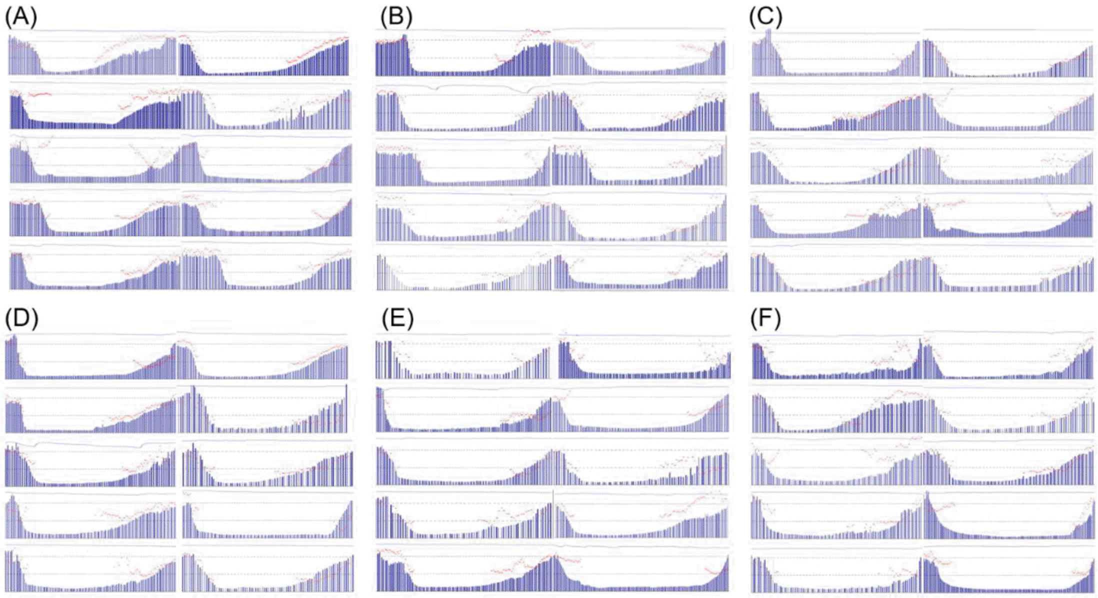


Figure 1

**INTERNATIONAL SEARCH REPORT**

International application No.
**PCT/CN2024/119212**

**A. CLASSIFICATION OF SUBJECT MATTER**

A61K31/724(2006.01)i; A61K9/19(2006.01)i; A61K9/08(2006.01)i; A61P21/00(2006.01)i; A61K31/27(2006.01)i

According to International Patent Classification (IPC) or to both national classification and IPC

**B. FIELDS SEARCHED**

Minimum documentation searched (classification system followed by classification symbols)

IPC: A61K 31/-; A61K 9/-; A61P 21/-

Documentation searched other than minimum documentation to the extent that such documents are included in the fields searched

Electronic data base consulted during the international search (name of data base and, where practicable, search terms used)

CNTXT, ENTXTC, DWPI, CNKI, ISI Web of Science, CAPLUS, REGISTRY: 奥默, 奥美克松, 环糊精, 苄异喹啉, 苄基异喹啉, 阿曲库铵, 新斯的明, 普洛巴林, 普洛斯的明, 逆转, 反转, 拮抗, 肌松, 肌肉, aom0498, adamgammadex, 1309580-40-2, 1309580-41-3, revers+, neuromuscular, neostigmine, prostigmin, proserin, +atracurium, benzylisoquinolines, +cyclodextrin?

**C. DOCUMENTS CONSIDERED TO BE RELEVANT**

| Category* | Citation of document, with indication, where appropriate, of the relevant passages | Relevant to claim No. |
|---|---|---|
| Y | CN 102060941 A (QI YOUMAO) 18 May 2011 (2011-05-18)<br>claim 5, and description, embodiments 9 and 27-28 | 11-12 |
| A | CN 102060941 A (QI YOUMAO) 18 May 2011 (2011-05-18)<br>claim 5, and description, embodiments 9 and 27-28 | 1-6 |
| Y | CN 1402737 A (AKZO NOBEL N.V.) 12 March 2003 (2003-03-12)<br>description, page 1 last paragraph-page 2 paragraph 1 | 11-12 |
| A | CN 1402737 A (AKZO NOBEL N.V.) 12 March 2003 (2003-03-12)<br>abstract, and claims 1-11 | 1-6 |
| X | WO 2023123468 A1 (HANGZHOU ADAMERCK PHARMLABS INC.) 06 July 2023 (2023-07-06)<br>claim 5, description, page 12 table 1, and pages 26-27 embodiment 11 | 7-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 December 2024** | **30 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/119212**

**C. DOCUMENTS CONSIDERED TO BE RELEVANT**

| Category* | Citation of document, with indication, where appropriate, of the relevant passages | Relevant to claim No. |
|---|---|---|
| A | STÄUBLE, C. G. et al. "The Future of Neuromuscular Blocking Agents" *Current Opinion in Anaesthesiology,* Vol. 33, No. 4, 31 August 2020 (2020-08-31), 490-498 ISSN: 0952-7907, page 490, abstract, page 494, right column, last paragraph-page 495, left column, paragraph 1 | 1-6 |
| A | JIANG, Yingying et al. "Comparison of the Efficacy and Safety of Adamgammadex with Sugammadex for Reversal of Rocuronium-Induced Neuromuscular Block: Results of a Phase II Clinical Trial" *Journal of Clinical Medicine,* Vol. 11, No. 23, 25 November 2022 (2022-11-25), 6951 (1-11) ISSN: 2077-0383, page 6951 (1/11), abstract | 1-6 |

Form PCT/ISA/210 (second sheet) (July 2022)

**INTERNATIONAL SEARCH REPORT**
**Information on patent family members**

International application No.
**PCT/CN2024/119212**

| Patent document cited in search report | Publication date (day/month/year) | Patent family member(s) | Publication date (day/month/year) |
|---|---|---|---|
| CN 102060941 A | 18 May 2011 | EP 2644624 A1 | 02 October 2013 |
| | | EP 2644624 A4 | 04 June 2014 |
| | | EP 2644624 B1 | 22 February 2017 |
| | | DK 2644624 T3 | 01 May 2017 |
| | | WO 2012068981 A1 | 31 May 2012 |
| | | JP 2013543915 A | 09 December 2013 |
| | | JP 5845279 B2 | 20 January 2016 |
| | | CA 2819007 A1 | 31 May 2012 |
| | | CA 2819007 C | 05 January 2016 |
| | | US 2013244979 A1 | 19 September 2013 |
| | | US 10017584 B2 | 10 July 2018 |
| | | US 2019010255 A1 | 10 January 2019 |
| | | US 10669352 B2 | 02 June 2020 |
| | | ES 2622332 T3 | 06 July 2017 |
| | | CN 102060941 B | 26 December 2012 |
| CN 1402737 A | 12 March 2003 | IL 149423 A0 | 10 November 2002 |
| | | HK 1049489 A1 | 16 May 2003 |
| | | CZ 20021814 A3 | 14 August 2002 |
| | | CZ 298206 B6 | 18 July 2007 |
| | | SK 7262002 A3 | 06 November 2002 |
| | | SK 286282 B6 | 06 June 2008 |
| | | JP 2006348301 A | 28 December 2006 |
| | | JP 4563972 B2 | 20 October 2010 |
| | | LU 91501 I2 | 26 January 2009 |
| | | LU 91501 I9 | 02 January 2019 |
| | | MXPA 02004940 A | 23 May 2003 |
| | | HUP 0203755 A2 | 28 April 2003 |
| | | HUP 0203755 A3 | 28 October 2003 |
| | | HU 227451 B1 | 28 June 2011 |
| | | TWI 242015 B | 21 October 2005 |
| | | BE vol. 2008C047 I2 | 11 December 2019 |
| | | AU 5438001 A | 12 June 2001 |
| | | AU 776536 B2 | 16 September 2004 |
| | | RU 2260013 C2 | 10 September 2005 |
| | | CY 2008018 I1 | 04 November 2009 |
| | | CO 5251450 A1 | 28 February 2003 |
| | | AR 026605 A1 | 19 February 2003 |
| | | BRPI 0015947 A | 20 August 2002 |
| | | BRPI 0015947 B1 | 07 February 2012 |
| | | BRPI 0015947 B8 | 06 July 2021 |
| | | JP 2003515623 A | 07 May 2003 |
| | | JP 3880041 B2 | 14 February 2007 |
| | | ES 2237496 T3 | 01 August 2005 |
| | | DE 122008000068 I1 | 23 April 2009 |
| | | DE 122008000068 I2 | 16 June 2011 |
| | | PL 356097 A1 | 14 June 2004 |
| | | PL 203012 B1 | 31 August 2009 |
| | | EP 1259550 A1 | 27 November 2002 |
| | | EP 1259550 B1 | 02 February 2005 |
| | | PE 20010902 A1 | 06 September 2001 |

Form PCT/ISA/210 (patent family annex) (July 2022)

**INTERNATIONAL SEARCH REPORT**
**Information on patent family members**

International application No.
**PCT/CN2024/119212**

| Patent document cited in search report | Publication date (day/month/year) | Patent family member(s) | Publication date (day/month/year) |
|---|---|---|---|
| | | NO 2010019 I1 | 22 November 2010 |
| | | NO 2010019 I2 | 27 August 2012 |
| | | NL 300356 I1 | 01 October 2008 |
| | | NL 300356 I2 | 01 April 2009 |
| | | DK 1259550 T3 | 25 April 2005 |
| | | ATE 288450 T1 | 15 February 2005 |
| | | FR 08C0052 I1 | 27 February 2009 |
| | | FR 08C0052 I2 | 18 December 2009 |
| | | US 6670340 B1 | 30 December 2003 |
| | | CA 2390463 A1 | 07 June 2001 |
| | | CA 2390463 C | 03 February 2009 |
| | | NO 20022522 D0 | 28 May 2002 |
| | | NO 20022522 L | 18 May 2010 |
| | | NZ 518752 A | 28 March 2003 |
| | | US 2004029833 A1 | 12 February 2004 |
| | | US 6949527 B2 | 27 September 2005 |
| | | KR 20020063900 A | 05 August 2002 |
| | | KR 100716524 B1 | 10 May 2007 |
| | | PT 1259550 E | 31 May 2005 |
| | | DE 60017947 D1 | 10 March 2005 |
| | | DE 60017947 T2 | 23 June 2005 |
| | | WO 0140316 A1 | 07 June 2001 |
| | | ZA 200203538 B | 04 August 2003 |
| | | USRE 44733 E | 28 January 2014 |
| | | NO 328790 B1 | 18 May 2010 |
| | | CN 1188428 C | 09 February 2005 |
| | | CY 200801812 I2 | 04 November 2009 |
| WO 2023123468 A1 | 06 July 2023 | EP 4458362 A1 | 06 November 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **H. D. DE BOER et al.** *British Journal of Anaesthesia*, 2006